# EUROPÄISCHE PATENTSCHRIFT

(11) **EP 4 297 568 B1**
(45) Veröffentlichungstag und Bekanntmachung des Hinweises auf die Patenterteilung: **02.07.2025**
(21) Anmeldenummer: 22710342.1
(22) Anmeldetag: 23.02.2022
(51) Int. Cl.: A01K 67/362

(54) **MODULE EINER AUFZUCHTSTATION FÜR INSEKTEN, VERFAHREN UND VERWENDUNG**
MODULE OF A BREEDING STATION FOR INSECTS, METHOD AND USE
MODULE D'UNE STATION D'ÉLEVAGE POUR INSECTES, MÉTHODE ET UTILISATION

(30) Priorität: 24.02.2021 EP 21159112
(43) Veröffentlichungstag der Anmeldung: 03.01.2024
(73) Patentinhaber: Illucens GmbH, 48683 Ahaus (DE)
(72) Erfinder: WESSENDORF, Dirk, 48683 Ahaus (DE)
(74) Vertreter: Taruttis, Tilman
(86) Internationale Anmeldenummer: PCT/EP2022/054582
(87) Internationale Veröffentlichungsnummer: WO 2022/180132

(56) Entgegenhaltungen:
- EP-A2- 0 724 828
- WO-A1-2014/171829
- WO-A1-2020/246878
- CN-A- 110 178 797
- CN-U- 209 643 626
- FR-A1- 3 046 333
- KR-B1- 102 024 635
- KR-B1- 102 190 437

## Beschreibung

In den bisher bekannten Systemen werden in Aufzuchtstationen für Insekten Insektenlarven in Behältnissen verschiedener Ausmaße gehalten. Zu diesem Zweck wird eine Kunststoffkiste mit einer definierten Menge Futter befüllt und anschließend die Junglarven auf dieses Futtergemisch gegeben. Für die nun anstehende Mastzeit müssen die Tiere unter klimatisch kontrollierten Bedingungen für einen Zeitraum von 6-10 Tagen, welches für das Insekt der Gattung "hermetia illucens" typisch ist, aufbewahrt werden. Nach Ablauf der Mastzeit werden diese Kisten manuell oder automatisiert dem Bereich entnommen und müssen nun geleert (ausgekippt) und die Larven von Kot und Futterresten getrennt werden.

Eine Trockensiebanlage ist zwingend erforderlich, so dass der Inhalt der Kisten über die Mastzeit getrocknet ist. Ist dieses nicht der Fall so kann die Absiebung des feuchten Gemisches zu Verstopfungen der Siebe oder schlechter Reinigungsleistung führen. Im Stand der Technik haben sich einige Schwierigkeiten herausgestellt:
- Verklebungen in den Kisten müssen mit Wasser gelöst bzw. gewaschen werden
- Tiere können während der Mast aus den Kisten unkontrolliert entkommen, welches zu Störungen führen kann und/oder die Insekten schlüpfen und sind in der Anlage
- Hoher Anteil an beweglichen Komponenten. Diese können ausfallen oder durch kleine technische Störungen kann der gesamte Ablauf gestört werden
- Hoher Energieaufwand da jegliche Masse mehrfach bewegt werden muss
- Großer Anteil an händischen Tätigkeiten da viele Bereiche nicht aufeinander abgestimmt sind

Der Stand der Technik nach WO 2020/246878 A1 beschreibt Systeme und Verfahren zur Aufzucht von wirbellosen Tieren, bei denen eine Vielzahl von Kisten in einer Vielzahl von Stapeln angeordnet wird. FR 3 046 333 B1 betrifft einen Behälter zur Insektenzucht, dessen Seitenwände sich nach außen erweitern, so dass der Boden und ein unterer Teil des Behälters durch die offene Oberseite eines identischen Behälters eingeführt werden können. WO 2014/171829 A1 beschreibt ein Verfahren und System zur Zucht von Insekten unter Verwendung einer Vielzahl einzelner Kisten, wobei zumindest ein Teil jeder Kiste mit einem Substrat gefüllt ist, das Futtervorräte und unreife Phasen von Insekten enthält. CN 209 643 626 U bezieht sich auf Ausrüstung zur Zucht von Schwarzmeer-Stören, insbesondere eine Zuchtbox für Schwarzmeer-Stör-Eier und Larven.

Aufgabe der Erfindung ist es, mit der Erfindung die aus dem Stand der Technik verbundenen Nachteile zumindest teilweise zu überwinden. Insbesondere kann eine verbesserte Anordnung und/oder vereinfacht handhabbare Aufbewahrung von Insektenlarven geschaffen werden.

Die Aufgabe wird mit dem Gegenstand der unabhängigen Ansprüche gelöst. Vorteilhafte Ausgestaltungen ergeben sich aus den jeweiligen abhängigen Ansprüchen und der Beschreibung.

Gemäß einem Aspekt der Erfindung werden, insbesondere U-förmige, Module verwendet, um eine Anordnung zum Aufbewahren von Insekten, insbesondere Insektenlarven, für eine Aufzucht derselben zu schaffen.

Mittels der, insbesondere U-förmigen, Module, die eine Basisfläche und zwei Seitenflächen aufweisen können, kann eine Aufbewahrungsmöglichkeit in Form einer mehrere Ebenen aufweisenden regalförmigen Einrichtung für einen Bioreaktor aufgebaut werden. Es kann eine, insbesondere stationäre, Masteinheit mit klimatisch steuerbaren Bedingungen geschaffen werden, die in der Höhe beliebig erweiterbar sein kann und/oder auch in der Breite eine variable Gestaltung zulässt.

Gleich ausgestaltete Module liefern die Möglichkeit eines gleichmäßigen Musters einer Anordnung, die den speziellen Anforderungen einer Insektenzucht genügen kann.

Einfach ausgestaltete Module können für den Aufbau einer derartigen Anordnung verwendet werden, mit der ein angemessenes Niveau der biologischen Sicherheit und ein wirksamer Schutz gegen unkontrolliertes Entweichen der Insekten erreicht werden kann.

Insbesondere kann mittels der Module erreicht werden, dass die Insekten auf einem starren Boden, der unbeweglich in der Anordnung ist, insbesondere in einem Stadium der Larvenwachstums, angeordnet werden können.

Die Anordnung kann eine Vielzahl von stapelbaren (beispielsweise Nut-Feder System) Modulen, insbesondere U-förmigen Module, aufweisen. Die Module können hinsichtlich ihrer Länge angepasst werden.

Die Module können insbesondere aus Kunststoff gefertigt sein, was die Herstellung und/oder die Handhabung im Hinblick auf Veränderung und/oder Gewicht vereinfachen kann. Kunststoff ist einfach handhabbar und die Module können beispielsweise mit simplen Mitteln abgelängt und in eine gewünschte Länge gebracht werden.

Es kann vorgesehen sein, einen umbauten Raum, beispielsweise eine Halle, mit den Modulen zu bestücken, indem die Module auf Abmessungen der Halle angepasst werden. Hierzu kann vorgesehen sein, dass elektrische und/oder mechanische Anschlüsse nur an einer Seite einer Längserstreckung des Moduls (entlang einer Seitenwand des Moduls) vorhanden sind, so dass das Modul in der Länge von dem Ende gekürzt werden kann, welches von dem mit den Anschlüssen versehenen Ende beabstandet ist. Eine große Variabilität ist auf diese Weise realisierbar.

Das U-förmige Modul kann an den oberen Enden der Seitenwände, insbesondere in Längserstreckung über die gesamte Länge der Seitenwände, jeweils ein Element eines Verbindungspaares aufweisen, das mit einem entsprechend am Boden eines weiteren Moduls ausgebildeten Elements des Verbindungspaares zusammenwirken kann, um einen mechanischen Eingriff der beiden Module zueinander auszubilden. Der mechanische Eingriff kann in Form eines Formschlusses ausgestaltet sein, der in Richtung quer zur Längserstreckung der Seitenwand und/oder in Richtung der Längserstreckung der Seitenwand vorliegen kann. Durch einen möglichen Formschluss der Module untereinander, der durch die Verbindungselemente, beispielsweise in Form von Nut und Feder, realisiert werden kann, ist eine stabile Verbindung möglich, ohne weitere Verbindungsstreben oder ähnliche Elemente zu benötigen.

Der (vorderseitige) Zugang eines U-förmigen Moduls weist eine plattenförmige Fläche auf, in der mindestens eine Öffnung in Form einer in das Innere des Moduls öffnende Klappe zur Befüllung des Moduls ausgebildet ist.

Der (vorderseitige) Zugang kann mittels eines Kunststoffteils, welches insbesondere eingeschweißt werden kann, verschlossen sein. Das Kunststoffteil kann durch 2 runde Öffnungen im oberen Bereich durchstoßen sein. Eine dieser Öffnungen kann in der waagerechten Achse mittig und in der senkrechten Achse im oberen Drittel angeordnet sein. Diese Öffnung kann von innen mit der nach innen öffnenden Klappe versehen sein. Durch die Öffnung kann beispielsweise ein Regalbediengerät mit einem Austragkopf bzw. einem Rohr in das Innere stoßen und Futter/Wasser oder Insektenlarven in das Modul einbringen. Eine gegebenenfalls vorhandene zweite Öffnung kann dazu dienen, bei Bedarf kontrolliert Luft in den Schacht einzulassen.

Das rückseitige Ende des Moduls (Ausgang) kann einen pneumatisch hochklappbaren verschließbaren Aufsatz, der insbesondere auch aus Kunststoff gefertigt sein kann, aufweisen. Während des Mastzyklusses kann der Aufsatz hochgeklappt verbleiben und das Futter und die Insektenlarven können im Modul verbleiben. Durch eine gezielte Ausbildung der Form des Aufsatzes kann jedoch Abluft weiterhin ungehindert ausströmen.

Das Modul kann zu allen Seiten geschlossen sein und die Insektenlarven können ausschließlich am Ausgang (hinten) das Modul verlassen. Sollte durch Überfüllung oder andere Gegebenheiten eine Abwanderung einzelner Insektenlarven erfolgen, so können diese nach unten fallen und in einem Behältnis aufgefangen werden, um diese dem weiteren Verarbeitungsprozess zuzuführen.

Jedes der Module kann einen geschlossenen Boden, der mit seiner Unterseite eine Decke für ein weiteres Modul sein kann, wenn die Module gestapelt sind, zwei gegenüberliegende Seitenwände und eine Zuführungswand und eine Abführungswand aufweisen. Der Boden, die Decke, die beiden Seitenwände und die Zuführungswand umgeben den Raum dicht, d.h. ohne Lücke. In der Zuführungswand können Öffnungen vorgesehen sein, die mit einem Verschluss versehen sind, der nach innen öffnet bzw. nach außen vorgespannt ist, so dass die Larven von innen nicht durch die Öffnungen in der Zuführungswand herausgelangen können. Die Abführungswand kann eine nach außen öffnende Klappe, Tür, Verschluss oder dergleichen aufweisen oder aus dieser bzw. diesem bestehen. Im bestimmungsgemäßen Gebrauch mit Larven gefüllte Module können üblicherweise nur über die Abführungswand geleert werden. Nur über die Abführungswand können Larven nach außen gelangen.

Der Erfindungsgedanke, stationäre Module für die Aufnahme von Insekten zu verwenden und keine Insekten mit Behältern hin und her zubewegen, ermöglicht eine verbesserte Automatisierung einer Insektenzucht.

Die Verwendung von Modulen zum Aufbau von stationären Räumen bzw. Bioreaktoren, in denen die Insekten aufbewahrt werden können, erlaubt es, für jeden der Räume ein Kleinklima einzustellen, dass den Anforderungen der Larven genügen kann, ohne dass beispielsweise eine große Halle klimatisiert werden muss.

Als Material für die U-förmigen Module ist ein Kunststoff bevorzugt, da dieser ein geringes Eigengewicht mit einer ausreichenden Stabilität aufweisen kann und eine Wiederverwendung aufgrund guter Reinigungsmöglichkeit gegeben sein kann. Es kann bevorzugt sein, dass das U-förmige Modul eine Wabenstruktur zwischen zwei plattenförmigen Elementen für Boden und die beiden Seitenwände aufweist, so dass neben einer möglichen Stabilität auch eine gewisse Wärmedämmung in Form von in den Waben eingeschlossener Luft vorliegen kann.

Um optimale Mastbedingungen für die Larven zu schaffen, können einige oder alle der Module jeweils mit einer Fußbodenheizung, die insbesondere bedarfsgerecht ausgestaltet ist, ausgestattet sein. Der Eintrag von Wärme über den Boden ist wesentlich effektiver als die Luft zu erwärmen und dann mit zusätzlichem Austrocken des Futters arbeiten zu müssen. Die, insbesondere in Modulen, vorgesehene Fußbodenheizung für eine Aufzucht von Insekten kann die Erfindung alleine tragen, aber auch mit den weiteren beschriebenen Ausgestaltungen zwanglos kombiniert werden, um eine vorteilhafte Ausgestaltung zu erreichen.

Es kann eine vorbestimmte Temperatur, insbesondere mittels der Fußbodenheizung, eingestellt werden. Es kann auch vorgesehen sein, dass im oder am Boden oder in oder an den Seitenwänden mindestens ein Kühlkanal ausgebildet ist, mit dem der in dem Modul bzw. in dem durch das Modul gebildeten Raum vorhandene Inhalt abgekühlt werden kann, insbesondere wenn die Temperatur durch eine Eigenbewegung der Larven und/oder wegen Kompostierungseffekten steigen sollte.

Einige oder alle der Module können mindestens einen Sensor für eine Temperaturmessung und/oder eine Feuchtigkeitsmessung an den Innenseiten der Seitenwände, der Zuführungswand, der Abführungswand, der Oberseite des Bodens und/oder der Unterseite des Bodens aufweisen. Mittels einer Steuerung kann ein Signal des Sensors bzw. der Sensoren erfasst und die Temperatur und/oder die Feuchtigkeit ermittelt werden. Hierdurch kann nicht nur ein Klimaparameter in Form der Temperatur und/oder der Feuchtigkeit gesteuert werden, sondern sogar, sofern Heiz-, Kühl-, Befeuchtungs- und/oder Entfeuchtungseinrichtungen entsprechend angesteuert werden, geregelt werden. Eine Abweichung vom Soll-Klima in Form von Temperatur und/oder Feuchtigkeit kann mittels des oder der Sensoren erfasst und mittels der genannten Einrichtungen entgegengesteuert werden. Dies kann nicht nur zu einer Optimierung der Ausbeute führen, sondern es kann auch eine erhöhte Produktqualität mit geringen Schwankungen erzielt werden.

Anstelle der Zuführung von Wärme mittels einer Fußbodenheizung kann dem Raum auch erwärmte Luft zugeführt werden. Eine Zuführung von gekühlter Luft zur Abkühlung der Temperatur im Raum ist ebenso wie die Zuführung von kaltem Wasser oder einem Kältemittel (welches durch einen oder mehrere Kanäle im Boden und/oder den Seitenwänden fließen kann) möglich. Es ist auch möglich eine Peltier-Kühlung vorzusehen. Die Zuführung von warmer Luft, warmem Wasser, gekühlter Luft, gekühlter Luft und/oder einem anderen Medium ist über, insbesondere geregelte, Ventile möglich.

Nachdem Futter in das Modul eingebracht wurde, können die Insektenlarven in das gegebenenfalls erwärmte Futter geblasen werden und sofort mit dem Wachstum beginnen. Über einen Zeitraum von 6-15 Tagen werden die Larven klimatisch gesteuert aufgezogen und dann aktiv mit Wasser über das pneumatisch abgesenkte Endstück des Ausgangs des Moduls ausgespült. Das Endstück kann durch die Abführungswand gebildet sein. Danach kann der Zyklus der Mast erneut gestartet werden.

Es kann sich einer oder mehrere der folgenden Vorteile ergeben:
- Stationär, modular, an Gegebenheiten (z.B. Gebäudegeometrie) anpassbar
- Maximale Volumenausbeute durch kompakte Bauweise
- Beheizbar (Fußbodenheizung der einzelnen Schächte), klimatisch regelbar
- Futter und Larven werden zum Ort der Mast transportiert. Mastort ist ortsfest.
- Kein Verlust durch Entkommen der Larven (Larven können nur in Ernterichtung wandern und werden dann automatisch verarbeitet)

Gemäß einer bevorzugten Ausführungsform kann einer oder mehrere der folgenden Aspekte umgesetzt werden:
- Mast der Insektenlarven in einem Modul variabler Länge
- Modul als stapelbares U(-Profil) mit Nut- und Federsystem (Feder an den Schachtwänden, Nut an der Unterseite des Profils)
- Hohlkammerprofil zum Einbau einer Fußbodenheizung
- Eine Seite (vorderseitig) durch ein eingeklebtes Kunststoffteil verschlossen (3-seitig), der Deckel eines Moduls bildet den Boden des oben aufgesetzten nächsten Moduls und umgekehrt
- Zweite Seite (rückseitig) ist das Modul durch einen pneumatisch hoch- und runterfahrbaren, knickbaren Aufsatz geschlossen. 2 Metallschalen sind mit einer Gummimembran verklebt und dadurch klappbar

## Patentansprüche

1. Anordnung zur Aufbewahrung von Insekten, insbesondere Insektenlarven, in Zeilen und/oder Spalten, wobei, insbesondere U-förmige, Module nebeneinander und/oder übereinander angeordnet sind, wobei ein Modul eine, insbesondere rechteckförmige, Basisfläche und zwei gegenüberliegende, insbesondere rechteckförmige, sich in eine Richtung von der Basisfläche erstreckende Seitenflächen aufweist, und quer zu den Seitenflächen vorderseitig ein Zugang zur Zuführung von Insektenlarven, Futter und/oder Wasser in das Modul vorgesehen ist, und quer zu den Seitenflächen rückseitig ein Ausgang vorgesehen ist,
wobei der vorderseitige Zugang eine plattenförmige Fläche aufweist, in der mindestens eine Öffnung zur Befüllung des Moduls ausgebildet ist,
**dadurch gekennzeichnet, dass** die Öffnung eine in das Innere des Moduls öffnende Klappe ist.

2. Anordnung nach Anspruch 1, wobei die Module aus Kunststoff gefertigt sind.

3. Anordnung nach Anspruch 1 oder 2, wobei die Module für eine Stapelung und/oder Anordnung nebeneinander kopfseitig und bodenseitig für einen Eingriff miteinander ausgestaltet sind.

4. Anordnung Anspruch 3, wobei kopfseitig ein Element einer mechanischen Verbindung und bodenseitig ein Gegenelement der mechanischen Verbindung vorliegt.

5. Anordnung nach Anspruch 3 oder 4, wobei der Eingriff bzw. das Element und das Gegenelement als Feder-Nut-System ausgestaltet ist, und das Element bzw. das Gegenelement eine Feder bzw. eine Nut oder umgekehrt ist.

6. Anordnung nach einem der Ansprüche 3 bis 5, wobei der Eingriff bzw. das Element und das Gegenelement kopfseitig und bodenseitig an den Seitenflächen angeordnet ist.

7. Anordnung nach Anspruch 6, wobei die beiden Seitenflächen kopfseitig gleich oder unterschiedlich ausgebildet sind und/oder die beiden Seitenflächen bodenseitig gleich oder unterschiedlich ausgebildet sind.

8. Anordnung nach einem der Ansprüche 1 bis 7, wobei einige oder alle der Module eine Fußbodenheizung aufweisen.

9. Anordnung nach einem der Ansprüche 1 bis 8, wobei die das Innere des Moduls öffnende Klappe selbstverschließend ist.

10. Anordnung nach einem der Ansprüche 1 bis 9, wobei die Seitenflächen und der Zugang im Wesentlichen luftdicht ausgestaltet sind.

11. Anordnung nach einem der Ansprüche 1 bis 10, wobei der Ausgang einen Verschluss aufweist, der um eine Verbindungslinie der Seitenflächen verschwenkbar ist, vorzugsweise nach unten zur rückseitigen Öffnung des Moduls.

12. Anordnung nach Anspruch 11, wobei der Verschluss pneumatisch hochklappbar ist.

13. Anordnung nach einem der Ansprüche 1 bis 12, wobei die Bodenfläche zumindest teilweise hohl ausgebildet ist.

14. Verfahren zum Aufbauen einer Anordnung zur Aufbewahrung von Insekten, insbesondere Insektenlarven, in Zeilen und/oder Spalten, insbesondere nach einem der Ansprüche 1 bis 13, wobei, insbesondere U-förmige, Module bereitgestellt werden, die nebeneinander und/oder übereinander angeordnet werden, wobei ein Modul eine, insbesondere rechteckförmige, Basisfläche und zwei gegenüberliegende, insbesondere rechteckförmige, sich in eine Richtung von der Basisfläche erstreckende Seitenflächen aufweist, und quer zu den Seitenflächen vorderseitig ein Zugang zur Zuführung von Insektenlarven, Futter und/oder Wasser in das Modul vorgesehen ist, und quer zu den Seitenflächen rückseitig ein Ausgang vorgesehen ist, wobei der vorderseitige Zugang eine plattenförmige Fläche aufweist, in der mindestens eine Öffnung zur Befüllung des Moduls ausgebildet ist, und wobei die Öffnung eine in das Innere des Moduls öffnende Klappe ist.

15. Verwendung von Modulen zum Aufbauen einer Anordnung zur Aufbewahrung von Insekten, insbesondere Insektenlarven, in Zeilen und/oder Spalten, insbesondere nach einem der Ansprüche 1 bis 13, wobei mehrere, insbesondere U-förmige, Module nebeneinander und/oder übereinander angeordnet sind, wobei ein Modul eine, insbesondere rechteckförmige, Basisfläche und zwei gegenüberliegende, insbesondere rechteckförmige, sich in eine Richtung von der Basisfläche erstreckende Seitenflächen aufweist, und quer zu den Seitenflächen vorderseitig ein Zugang zur Zuführung von Insektenlarven, Futter und/oder Wasser in das Modul vorgesehen ist, und quer zu den Seitenflächen rückseitig ein Ausgang vorgesehen ist, wobei der vorderseitige Zugang eine plattenförmige Fläche aufweist, in der mindestens eine Öffnung zur Befüllung des Moduls ausgebildet ist, und wobei die Öffnung eine in das Innere des Moduls öffnende Klappe ist.

## Claims

1. Arrangement for storing insects, in particular insect larvae, in rows and/or columns, wherein in particular U-shaped modules are arranged next to one another and/or above one another, wherein a module has an in particular rectangular base surface and two opposite, in particular rectangular, side surfaces extending in a direction from the base surface, and an entry for supplying insect larvae, food and/or water into the module is provided transversely to the side surfaces at the front, and an exit is provided transversely to the side surfaces at the rear,
wherein the front entry has a planar surface in which at least one opening for filling the module is formed,
**characterized in that** the opening is a flap which opens into the interior of the module.

2. Arrangement according to claim 1, wherein the modules are made of plastics material.

3. Arrangement according to claim 1 or 2, wherein the modules, for being stacked and/or arranged next to one another, are designed to engage with one another at the top and the bottom.

4. Arrangement according to claim 3, wherein an element of a mechanical connection is present at the top and a mating element of the mechanical connection is present at the bottom.

5. Arrangement according to claim 3 or 4, wherein the engagement, or the element and the mating element, is designed as a tongue-and-groove system, and the element and mating element is a tongue and groove, or vice versa.

6. Arrangement according to any of claims 3 to 5, wherein the engagement, or the element and the mating element, is arranged at the top and bottom of the side surfaces.

7. Arrangement according to claim 6, wherein the two side surfaces are of the same or different design at the top and/or the two side surfaces are of the same or different design at the bottom.

8. Arrangement according to any of claims 1 to 7, wherein some or all of the modules have underfloor heating.

9. Arrangement according to any of claims 1 to 8, wherein the flap opening the interior of the module is self-closing.

10. Arrangement according to any of claims 1 to 9, wherein the side surfaces and the entry are designed to be substantially airtight.

11. Arrangement according to any of claims 1 to 10, wherein the exit has a closure which is pivotable about a connecting line of the side surfaces, preferably downwards towards the rear opening of the module.

12. Arrangement according to claim 11, wherein the closure is pneumatically upwardly foldable.

13. Arrangement according to any of claims 1 to 12, wherein the bottom surface is at least partially hollow.

14. Method for constructing an arrangement for storing insects, in particular insect larvae, in rows and/or columns, in particular according to any of claims 1 to 13, wherein in particular U-shaped modules are provided which are arranged next to one another and/or above one another, wherein a module has an in particular rectangular base surface and two opposite, in particular rectangular, side surfaces extending in a direction from the base surface, and an entry for supplying insect larvae, food and/or water into the module is provided transversely to the side surfaces at the front, and an exit is provided transversely to the side surfaces at the rear, wherein the front entry has a planar surface in which at least one opening for filling the module is formed, and wherein the opening is a flap which opens into the interior of the module.

15. Use of modules for constructing an arrangement for storing insects, in particular insect larvae, in rows and/or columns, in particular according to any of claims 1 to 13, wherein a plurality of in particular U-shaped modules are arranged next to one another and/or above one another, wherein a module has an in particular rectangular base surface and two opposite, in particular rectangular, side surfaces extending in a direction from the base surface, and an entry for supplying insect larvae, food and/or water into the module is provided transversely to the side surfaces at the front, and an exit is provided transversely to the side surfaces at the rear, wherein the front entry has a planar surface in which at least one opening for filling the module is formed, and wherein the opening is a flap which opens into the interior of the module.

## Revendications

1. Agencement permettant la conservation d'insectes, en particulier de larves d'insectes, dans des lignes et/ou des colonnes, dans lequel des modules, en particulier en forme de U, sont disposés les uns à côté des autres et/ou les uns au-dessus des autres, un module présentant une surface de base, en particulier rectangulaire, et deux surfaces latérales opposées, en particulier rectangulaires, s'étendant dans une direction à partir de la surface de base et, transversalement aux surfaces latérales, un accès est prévu du côté avant pour l'introduction de larves d'insectes, de nourriture et/ou d'eau dans le module et, transversalement aux surfaces latérales, une sortie est prévue du côté arrière,
l'accès du côté avant présentant une surface en forme de plaque dans laquelle est réalisée au moins une ouverture permettant le remplissage du module,
**caractérisé en ce que** l'ouverture est un clapet s'ouvrant vers l'intérieur du module.

2. Agencement selon la revendication 1, dans lequel les modules sont fabriqués en matière plastique.

3. Agencement selon la revendication 1 ou 2, dans lequel les modules sont conçus, pour un empilement et/ou un agencement côte à côte, pour une prise les uns avec les autres du côté de la tête et du côté du fond.

4. Agencement selon la revendication 3, dans lequel un élément d'une liaison mécanique est présent du côté de la tête et un contre-élément de la liaison mécanique est présent du côté du fond.

5. Agencement selon la revendication 3 ou 4, dans lequel la prise ou l'élément et le contre-élément sont conçus comme un système à rainure-languette et l'élément ou le contre-élément est une languette ou, selon le cas, une rainure ou inversement.

6. Agencement selon l'une des revendications 3 à 5, dans lequel la prise ou l'élément et le contre-élément sont disposés du côté de la tête et du côté du fond au niveau des surfaces latérales.

7. Agencement selon la revendication 6, dans lequel les deux surfaces latérales sont réalisées de manière identique ou différente du côté de la tête et/ou les deux surfaces latérales sont réalisées de manière identique ou différente du côté du fond.

8. Agencement selon l'une des revendications 1 à 7, dans lequel certains ou la totalité des modules présentent un chauffage par le fond.

9. Agencement selon l'une des revendications 1 à 8, dans lequel le clapet ouvrant l'intérieur du module est auto-obturant.

10. Agencement selon l'une des revendications 1 à 9, dans lequel les surfaces latérales et l'accès sont conçus de manière sensiblement étanches à l'air.

11. Agencement selon l'une des revendications 1 à 10, dans lequel la sortie présente un obturateur qui peut pivoter autour d'une ligne de jonction des surfaces latérales, de préférence vers le bas en direction de l'ouverture du côté arrière du module.

12. Agencement selon la revendication 11, dans lequel l'obturateur peut être relevé pneumatiquement.

13. Agencement selon l'une des revendications 1 à 12, dans lequel la surface de fond est réalisée de manière au moins partiellement creuse.

14. Procédé permettant la construction d'un agencement permettant la conservation d'insectes, en particulier de larves d'insectes, dans des lignes et/ou des colonnes, en particulier selon l'une des revendications 1 à 13, dans lequel des modules, en particulier en forme de U, sont mis à disposition, qui sont disposés les uns à côté des autres et/ou les uns au-dessus des autres, un module présentant une surface de base, en particulier rectangulaire, et deux surfaces latérales opposées, en particulier rectangulaires, s'étendant dans une direction à partir de la surface de base et, transversalement aux surfaces latérales, un accès est prévu du côté avant pour l'introduction de larves d'insectes, de nourriture et/ou d'eau dans le module et, transversalement aux surfaces latérales, une sortie est prévue du côté arrière, l'accès du côté avant présentant une surface en forme de plaque dans laquelle est réalisée au moins une ouverture permettant le remplissage du module et l'ouverture étant un clapet s'ouvrant vers l'intérieur du module.

15. Utilisation de modules permettant la construction d'un agencement permettant la conservation d'insectes, en particulier de larves d'insectes, dans des lignes et/ou des colonnes, en particulier selon l'une des revendications 1 à 13, dans laquelle plusieurs modules, en particulier en forme de U, sont disposés les uns à côté des autres et/ou les uns au-dessus des autres, un module présentant une surface de base, en particulier rectangulaire, et deux surfaces latérales opposées, en particulier rectangulaires, s'étendant dans une direction à partir de la surface de base et, transversalement aux surfaces latérales, un accès est prévu du côté avant pour l'introduction de larves d'insectes, de nourriture et/ou d'eau dans le module et, transversalement aux surfaces latérales, une sortie est prévue du côté arrière, l'accès du côté avant présentant une surface en forme de plaque dans laquelle est réalisée au moins une ouverture pour le remplissage du module et l'ouverture étant un clapet s'ouvrant vers l'intérieur du module.
